# EUROPEAN PATENT SPECIFICATION

(11) **EP 3 513 194 B1**
(45) Date of publication and mention of the grant of the patent: **01.06.2022**
(21) Application number: 17851704.1
(22) Date of filing: 18.09.2017
(51) Int. Cl.: G01N 33/58

(54) **METHODS AND SYSTEMS FOR SCREENING CANDIDATE COMPOUNDS FOR THEIR POTENTIAL TO CAUSE SYSTEMIC OR HEPATIC TOXICITY**
VERFAHREN UND SYSTEME ZUM SCREENING VON KANDIDATENVERBINDUNGEN AUF IHR POTENZIAL, SYSTEMISCHE ODER HEPATISCHE TOXIZITÄT ZU VERURSACHEN
PROCÉDÉS ET SYSTÈMES DE DÉPISTAGE DE COMPOSÉS CANDIDATS À LA PROVOCATION POTENTIELLE D'UNE TOXICITÉ SYSTÉMIQUE OU HÉPATIQUE

(30) Priority: 16.09.2016 US 201662395503 P
(43) Date of publication of application: 24.07.2019
(73) Proprietor: Qualyst Transporter Solutions LLC, Durham, NC 27713 (US)
(72) Inventor: BROUWER, Kenneth, R., Durham, NC 27713 (US); JACKSON, Jonathan, P., Durham, NC 27713 (US); BLACK, Christopher, B., Durham, NC 27713 (US)
(74) Representative: Jilderda, Anne Ayolt
(86) International application number: PCT/US2017/052022
(87) International publication number: WO 2018/053406

(56) References cited:
- EP-A1- 2 762 573
- WO-A1-2016/057626
- US-A1- 2010 179 798
- US-B2- 8 658 353
- TRACY L. MARION ET AL: "Differential Disposition of Chenodeoxycholic Acid versus Taurocholic Acid in Response to Acute Troglitazone Exposure in Rat Hepatocytes", TOXICOLOGICAL SCIENCES, vol. 120, no. 2, 24 January 2011 (2011-01-24), pages 371-380, XP055670525, ISSN: 1096-6080, DOI: 10.1093/toxsci/kfr014
- KYUNGHEE YANG ET AL: "Species Differences in Hepatobiliary Disposition of Taurocholic Acid in Human and Rat Sandwich-Cultured Hepatocytes: Implications for Drug-Induced Liver Injury", JOURNAL OF PHARMACOLOGY AND EXPERIMENTAL THERAPEUTICS, vol. 353, no. 2, 6 May 2015 (2015-05-06), pages 415-423, XP055670549, US ISSN: 0022-3565, DOI: 10.1124/jpet.114.221564
- JONATHAN P. JACKSON ET AL: "Cholestatic Drug Induced Liver Injury: A Function of Bile Salt Export Pump Inhibition and Farnesoid X Receptor Antagonism", APPLIED IN VITRO TOXICOLOGY, vol. 4, no. 3, 1 September 2018 (2018-09-01), pages 265-279, XP055670524, ISSN: 2332-1512, DOI: 10.1089/aivt.2018.0011
- OGIMURA et al.: "Bile salt export pump inhibitors are associated with bile acid-dependent drug-induced toxicity in sandwich-cultured hepatocytes", Biochemical and Biophysical Research Communications, vol. 416, no. 3-4, December 2011 (2011-12) , pages 313-317, XP028342317,
- YANG et al.: "Sandwich-Cultured Hepatocytes as a Tool to Study Drug Disposition and Drug-Induced Liver Injury", Journal of Pharmaceutical Sciences, vol. 105, no. 2, February 2016 (2016-02), pages 443-459, XP055595188, DOI: 10.1016/j.xphs.2015.11.008
- SOROKA et al.: "Mouse organic solute transporter alpha deficiency enhances renal excretion of bile acids and attenuates cholestasis", Hepatology, vol. 51, no. 1, January 2010 (2010-01), XP055489508,
- ANSEDE et al.: "An In Vitro Assay to Assess Transporter-Based Cholestatic Hepatotoxicity Using Sandwich- Cultured Rat Hepatocytes", Drug Metabolism And Disposition, vol. 38, no. 2, February 2010 (2010-02), pages 276-280, XP055416719,
- LEFEBVRE et al.: "Role of Bile Acids and Bile Acid Receptors in Metabolic Regulation", Physiological Reviews, vol. 89, no. 1, January 2009 (2009-01), pages 147-191, XP055489509,
- WOODHEAD et al.: "Mechanistic Modeling Reveals the Critical Knowledge Gaps in Bile Acid-Mediated DILI", CPT Pharmacometrics Systems Pharmacology, vol. 3, no. 7, July 2014 (2014-07), pages 1-8, XP055489511,
- James L. Boyer ET AL: "Upregulation of a basolateral FXR-dependent bile acid efflux transporter OST[alpha]-OST[beta] in cholestasis in humans and rodents", American Journal of Physiology - Gastrointestinal and Liver Physiology, vol. 290, no. 6, 1 June 2006 (2006-06-01), pages G1124-G1130, XP055766883, US ISSN: 0193-1857, DOI: 10.1152/ajpgi.00539.2005
- R. Kaimal ET AL: "Differential Modulation of Farnesoid X Receptor Signaling Pathway by the Thiazolidinediones", Journal of Pharmacology and Experimental Therapeutics, vol. 330, no. 1, 1 July 2009 (2009-07-01), pages 125-134, XP055425553, US ISSN: 0022-3565, DOI: 10.1124/jpet.109.151233

## Description

### CROSS-REFERENCE TO RELATED APPLICATIONS

This application claims benefit of U.S. Provisional Patent Application Serial No. 62/395,503, filed September 16, 2016.

### TECHNICAL FIELD

The instant disclosure is directed to screening candidate compounds or chemical entities for their cholestatic hepatotoxicity potential. More particularly, the instant disclosure is directed to methods and systems for screening candidate compounds for susceptibility to causing, or their potential to cause, systemic or hepatic toxicity.

### BACKGROUND

In developing new therapeutics, drugs and pharmaceutical compounds there is a need to screen new chemical entities (NCE) to determine their cholestatic hepatotoxicity potential. Drug induced cholestatic hepatotoxicity is the result of impaired bile flow of bile acids resulting in the accumulation of toxic concentrations of bile acids in the hepatocyte. NCEs that are likely to cause drug induced cholestatic hepatotoxicity may be removed from consideration in further drug development trials.

There is a need for improved *in vitro* methodologies and systems to assess the potential of a NCE, compound or drug candidate to cause cholestatic hepatotoxicity *in vivo.* Such needs are addressed by the presently disclosed subject matter.

### SUMMARY

The invention is defined in the appended claims. This summary lists several embodiments of the presently disclosed subject matter, and in many cases lists variations and permutations of these embodiments. This summary is merely exemplary of the numerous and varied embodiments. Mention of one or more representative features of a given embodiment is likewise exemplary. Such an embodiment can typically exist with or without the feature(s) mentioned; likewise, those features can be applied to other embodiments of the presently disclosed subject matter, whether listed in this summary or not. To avoid excessive repetition, this Summary does not list or suggest all possible combinations of such features.

Provided herein are methods of screening a compound for its potential to cause systemic and/or hepatic toxicity, the methods comprising the steps of: providing a compound to be screened, establishing a hepatic cell system (HCS) comprising a capacity for bile acid synthesis, bile acid transport and/or bile acid regulation, exposing the HCS to a range of concentrations of a bile acid to determine a toxicity profile of the bile acid, wherein the bile acid toxicity profile comprises a toxicity potency, exposing the HCS to a range of concentrations of a bile acid in the presence of the compound to be screened and determining a toxicity profile of the bile acid, wherein the bile acid toxicity profile comprises a toxicity potency, and comparing the toxicity potencies of the bile acids to determine the susceptibility of the compound to cause systemic and/or hepatic toxicity.

Provided herein are *in vitro* systems for predicting *in vivo* hepatotoxic potential of a compound, comprising *in vitro* cultured HCS comprising a capacity for bile acid synthesis, bile acid transport and/or bile acid regulation, one or more bile acids with an established toxicity potency within the HCS, and an assay for determining the hepatotoxicity of a compound when exposed to the HCS in the presence of the one or more bile acids.

Provided herein are methods of screening a compound for susceptibility to causing systemic and/or hepatic toxicity, the methods comprising providing a compound to be screened, establishing a HCS comprising a capacity for bile acid synthesis, bile acid transport and/or bile acid regulation, exposing the HCS to a range of concentrations of a bile acid in the presence of the compound to be screened and determining a toxicity profile of the bile acid in the presence of the compound to be screened, wherein the toxicity profile of the bile acid is known across the range of concentrations, wherein the toxicity profile of the bile acid comprises a toxicity potency of the bile acid, and comparing the toxicity potency of the bile acid in the presence and absence of the compound to determine the potential of the compound to cause systemic and/or hepatic toxicity.

Accordingly, it is an object of the presently disclosed subject matter to provide methods and systems for screening a compound for susceptibility to causing systemic and/or hepatic toxicity. This and other objects are achieved in whole or in part by the presently disclosed subject matter. Further, an object of the presently disclosed subject matter having been stated above, other objects and advantages of the presently disclosed subject matter will become apparent to those skilled in the art after a study of the following description, Drawings and Examples.

### BRIEF DESCRIPTION OF THE DRAWINGS

The presently disclosed subject matter can be better understood by referring to the following figures. The components in the figures are not necessarily to scale, emphasis instead being placed upon illustrating the principles of the presently disclosed subject matter (often schematically). In the figures, like reference numerals designate corresponding parts throughout the different views. A further understanding of the presently disclosed subject matter can be obtained by reference to an embodiment set forth in the illustrations of the accompanying drawings. Although the illustrated embodiment is merely exemplary of systems for carrying out the presently disclosed subject matter, both the organization and method of operation of the presently disclosed subject matter, in general, together with further objectives and advantages thereof, may be more easily understood by reference to the drawings and the following description. The drawings are not intended to limit the scope of this presently disclosed subject matter, which is set forth with particularity in the claims as appended or as subsequently amended, but merely to clarify and exemplify the presently disclosed subject matter.

For a more complete understanding of the presently disclosed subject matter, reference is now made to the following drawings in which:
Figures 1A and 1B are schematic illustrations of hepatocytes showing normal bile acid homeostatic pathways *in vivo* (Figure 1A), versus compromised bile acid homeostasis potentially leading to drug induced cholestatic hepatotoxicity (Figure 1B);
Figure 2 is a schematic illustration of hepatocytes showing normal bile acid bile acid uptake, synthesis and export *in vivo*;
Figure 3 is a schematic illustration of hepatocytes showing effects of a NCE acting as a BSEP inhibitor initiating the bile acid homeostasis feedback mechanism resulting in the induction of the compensatory mechanism and lowering of the intracellular concentrations of bile acids;
Figure 4 is a schematic illustration of hepatocytes showing effects of a NCE acting as a BSEP inhibitor and FXR antagonist preventing the activation of the bile acid homeostasis feedback mechanism resulting in increased bile acid intracellular concentrations leading to bile acid hepatotoxicity;
Figure 5 is a schematic illustration of hepatocytes showing effects of a NCE inhibiting multiple bile acid efflux routes, resulting in hepatotoxicity;
Figure 6 is a schematic illustration of hepatocytes showing effects of a NCE inhibiting bile acid uptake, resulting in reducing the amount of bile acids presented to the hepatocyte leading to systemic cholestasis;
Figures 7 and 8 are graphical depictions of the effects of increasing bile acid in the presence of NCE on ATP content (Figure 7) and LDH leakage (Figure 8);
Figures 9 and 10 are scatter plots showing the results of increasing concentrations of bile acids GCA, GCDCA and GDCA in SCHH on ATP (Figure 9) and LDH (Figure 10);
Figures 11 and 12 are plots showing the results of glucose concentrations (5 mM glucose, Figure 11; 11 mM glucose, Figure 12) on bile acid pool toxicity profiles in the absence and presence of cholestatic agents in SCHH;
Figure 13 is a plot showing the data from a feasibility study assessing the ability of the disclosed assays, methods and systems to distinguish Ambrisentan from Sitaxsentan;
Figures 14 through 18 are plots showing the data from a viability studies based on exposure to bile acids and bile acid pools;
Figures 19 through 21 are plots showing the data from evaluations of the effects of free fatty acids on the hepatotoxicity of the bile acid pool in the presence of troglitazone;
Figures 22A through 24B are histograms showing the data from studies evaluating the susceptibility of hepatocytes to bile acid toxicity when drug exposure inhibits bile acid efflux routes;
Figures 25A and 25B are dot plots showing the results of inhibition of biliary clearance of d8-TCA (Figure 25A) and biliary efflux of d8-TCA (Figure 25B) observed in SCHH cultures in the presence of troglitazone;
Figures 26A and 26B are histograms demonstrating FXR antagonism in SCHH following exposure to troglitazone;
Figure 27 is a histogram of ATP content and LDH leakage evaluating cell viability under the conditions utilized to examine FXR antagonism;
Figures 28A and 28B are histograms showing the effects SCHH treated with troglitazone, pioglitazone, and rosiglitazone under sensitization culture conditions on LDH leakage and loss of ATP content.

### DETAILED DESCRIPTION

The presently disclosed subject matter now will be described more fully hereinafter, in which some, but not all embodiments of the presently disclosed subject matter are described. Indeed, the presently disclosed subject matter can be embodied in many different forms and should not be construed as limited to the embodiments set forth herein; rather, these embodiments are provided so that this disclosure will satisfy applicable legal requirements.

The terminology used herein is for the purpose of describing particular embodiments only and is not intended to be limiting of the presently disclosed subject matter.

While the following terms are believed to be well understood by one of ordinary skill in the art, the following definitions are set forth to facilitate explanation of the presently disclosed subject matter.

All technical and scientific terms used herein, unless otherwise defined below, are intended to have the same meaning as commonly understood by one of ordinary skill in the art. References to techniques employed herein are intended to refer to the techniques as commonly understood in the art, including variations on those techniques or substitutions of equivalent techniques that would be apparent to one of skill in the art. While the following terms are believed to be well understood by one of ordinary skill in the art, the following definitions are set forth to facilitate explanation of the presently disclosed subject matter.

In describing the presently disclosed subject matter, it will be understood that a number of techniques and steps are disclosed. Each of these has individual benefit and each can also be used in conjunction with one or more, or in some cases all, of the other disclosed techniques.

Accordingly, for the sake of clarity, this description will refrain from repeating every possible combination of the individual steps in an unnecessary fashion. Nevertheless, the specification and claims should be read with the understanding that such combinations are entirely within the scope of the invention and the claims.

Following long-standing patent law convention, the terms "a", "an", and "the" refer to "one or more" when used in this application, including the claims. Thus, for example, reference to "a cell" includes a plurality of such cells, and so forth.

Unless otherwise indicated, all numbers expressing quantities of ingredients, reaction conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed subject matter.

As used herein, the term "about," when referring to a value or to an amount of a composition, dose, sequence identity (e.g., when comparing two or more nucleotide or amino acid sequences), mass, weight, temperature, time, volume, concentration, percentage, *etc.*, is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed methods or employ the disclosed compositions.

The term "comprising", which is synonymous with "including" "containing" or "characterized by" is inclusive or open-ended and does not exclude additional, unrecited elements or method steps. "Comprising" is a term of art used in claim language which means that the named elements are essential, but other elements can be added and still form a construct within the scope of the claim.

As used herein, the phrase "consisting of" excludes any element, step, or ingredient not specified in the claim. When the phrase "consists of" appears in a clause of the body of a claim, rather than immediately following the preamble, it limits only the element set forth in that clause; other elements are not excluded from the claim as a whole.

As used herein, the phrase "consisting essentially of" limits the scope of a claim to the specified materials or steps, plus those that do not materially affect the basic and novel characteristic(s) of the claimed subject matter.

With respect to the terms "comprising", "consisting of", and "consisting essentially of", where one of these three terms is used herein, the presently disclosed and claimed subject matter can include the use of either of the other two terms.

As used herein, the term "and/or" when used in the context of a listing of entities, refers to the entities being present singly or in combination. Thus, for example, the phrase "A, B, C, and/or D" includes A, B, C, and D individually, but also includes any and all combinations and subcombinations of A, B, C, and D.

As used herein, a hepatic cell system (HCS) refers to a hepatic cell system comprising a capacity for bile acid synthesis, bile acid transport and/or bile acid regulation. In some embodiments, the HCS can comprise a two (2)-dimensional culture, such as but not limited to sandwich culture of hepatocytes (SCH), including a sandwich culture of human hepatocytes (SCHH). In some embodiments, the HCS can comprise a three (3)-dimensional culture (such as but not limited to 3D scaffold-based cultures, spheroidal cultures and the like. The HCS can comprise primary hepatocytes or other relevant hepatic cell systems, such as but not limited to HepaRG, Huh7, co-cultured systems, and/or stem-cell derived hepatocytes. In some embodiments, one or more free fatty acids and/or a preselected glucose concentration is/are employed in the HCS, such as in the incubation media of the HCS.

Disclosed herein are methods and systems for screening candidate compounds for susceptibility to causing, or their potential to cause, systemic or hepatic toxicity. As used herein, terms used to refer to a candidate compound's "ability", "susceptibility", "potential", "probability", "likelihood", or the like, are used interchangeably and are generally indicative of a likelihood that a compound can impact, cause or contribute to system or hepatic toxicity and related conditions as disclosed and discussed herein.

The presently disclosed subject matter provides in some embodiments *in vitro* methodologies and systems to assess the potential of a new chemical entity (NCE), compound or drug candidate to cause cholestatic hepatotoxicity *in vivo.* Primary bile acids are synthesized in the hepatocyte by one or more hepatic enzymes, including for example by Cyp7A1, and then rapidly conjugated to both taurine and glycine, and may be further altered through glucuronidation and sulfation. Bile acids and conjugates are excreted in the bile by the Bile Salt Export Protein (BSEP) and MRP2 and into the portal circulation by MRP3/4 or OSTα/β. Bile acids undergo further metabolism in the intestine and are absorbed in the enterocyte by ASBT, and effluxed from the enterocyte into the portal vein by OSTα/β. Bile acids are taken up from the portal circulation primarily by NTCP; however, some studies have also suggested that OATPs may be responsible for some amount of uptake. Bile acids can act as detergents, and high intracellular concentrations (ICC) of bile acids have been shown to be hepatotoxic.

Drug induced cholestatic hepatotoxicity is generally thought to be the result of impaired bile flow of bile acids resulting from inhibition of only BSEP by the NCE. However, impairment of the hepatocyte bile acid homeostasis adaptive response, mediated by the farnesoid X receptor (FXR), likely plays a pivotal role in the drug induced liver injury. Without being bound by any particular theory or mechanism of action and/or the current dogma, it is currently believed that inhibition of BSEP by the NCE is only the initiating event resulting in an increase in the intracellular concentrations of bile acids. When the intracellular concentration of bile acids is high enough, they activate FXR, which triggers a decrease in the expression of CYP7A1, the rate limiting enzyme in bile acid production, resulting in decreased bile acid synthesis and induction of both BSEP and OSTα/β expression. The increase in BSEP expression will have minimal effect since the NCE is inhibiting transport by that route; however, the induction of OSTα/β will greatly increase the bile acid clearance from the hepatocyte into the portal circulation, thereby allowing the hepatocyte to decrease the intracellular concentration of bile acids, and decrease the potential for hepatotoxicity. Therefore, the proposed mechanism for cholestatic hepatotoxicity is that a potential cholestatic hepatotoxicant must inhibit bile acid efflux routes (e.g. BSEP and/or MRP3/4) and 1) prevent (antagonize) FXR from sensing the rising intracellular concentrations of bile acids; or 2) inhibit bile acid efflux through OSTα/β, a bile acid efflux compensatory pathway or 3) a combination of both.

In order to accurately predict an *in vivo* effect on the hepatic disposition of bile acids, an integrated system as provided herein can be important, if not required. In some embodiments, the integrated system comprises synthesis, transport (uptake, basolateral efflux, and canalicular efflux), and regulation (transport, metabolism and synthesis). The sandwich-cultured human hepatocyte (SCHH) model is one system that is characterized and capable of combining all of these pathways.

The presently disclosed methods and systems combine the utility of the SCHH model for determination of BSEP inhibition and bile acid regulation through FXR. In addition, it was observed that the intracellular concentration of bile acids is the driving force for the activation of the FXR feedback mechanism. Since the intracellular concentration of bile acid is a factor for the development of an *in vitro* methodology to predict cholestatic hepatotoxicity, it was determined that the intracellular concentration (ICC) of bile acids was required to activate the FXR feedback mechanism. In addition, different bile acids are known to have different potentials for hepatotoxicity, therefore multiple bile acids and combinations thereof (bile acid pool) were evaluated for their potential to elicit a hepatotoxic response using standard assays for toxicity (for example, but not limited to, ATP, LDH, Caspase 3/7 and APOTOX Glo), and various mixtures of bile acids, including deoxycholic acid (DCA) identified as one of the most potent hepatotoxic bile acids. Use of these assays allowed for the differentiation between cell viability, necrosis and apoptosis in the system.

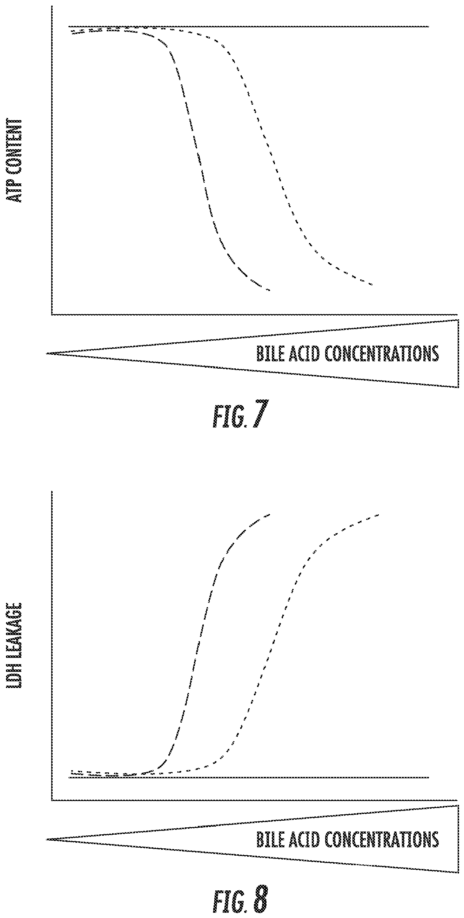

Generally, the presently disclosed methods can in some embodiments comprise culturing SCHH with a concentration range of a bile acid (e.g. DCA), or bile acid pool to identify the toxicity profile of the bile acid (such as by measurement of ATP and/or LDH; dotted line in Figures 7 and 8). In a parallel study, SCHH can be exposed to a concentration range of DCA or combination of bile acids in the presence of a potential cholestatic hepatotoxicant. Three possible outcomes could then be observed:
1. No change in BA toxicity potency in presence of NCE = No predicted effect;
2. Increase in BA toxicity potency in the presence of NCE = potential to cause cholestatic hepatotoxicity , e.g. a BA efflux inhibitor, FXR antagonist, or both; or
3. Decrease in BA toxicity potency in the presence of NCE = potential to cause cholestasis may result in systemic toxicity, e.g. a BA uptake inhibitor.

The NCE could then be classified as having no effect on bile acid homeostasis (1), or a high potential to cause cholestatic hepatotoxicity which may or may not be associated with increased systemic concentrations of bile acids (2), or having a potential for observation of clinical cholestasis (3) (increased systemic concentrations of bile acids with little potential for hepatic cholestasis), or a combination thereof.

Thus, in some embodiments, provided herein are methods of screening a compound for its potential to cause systemic and/or hepatic toxicity. In some embodiments, the methods comprise providing a compound to be screened, establishing a hepatic cell system (HCS) comprising a capacity for bile acid synthesis, bile acid transport and/or bile acid regulation, exposing the HCS to a range of concentrations of a bile acid to determine a toxicity profile of the bile acid, wherein the bile acid toxicity profile comprises a toxicity potency, exposing the HCS to a range of concentrations of a bile acid in the presence of the compound to be screened and determining a toxicity profile of the bile acid, wherein the bile acid toxicity profile comprises a toxicity potency, and comparing the toxicity potencies of the bile acids to determine the potential of the compound to cause systemic and/or hepatic toxicity.

In some aspects, determining the potential of the compound to cause systemic or hepatic toxicity further comprises identifying no change in the bile acid toxicity potency in the presence of the compound as compared to the absence of the compound, identifying an increase in the bile acid toxicity in the presence of the compound as compared to the absence of the compound, or identifying a decrease in the bile acid toxicity in the presence of the compound as compared to the absence of the compound. A compound causing no change in the bile acid toxicity potency is characterized as not causing systemic or hepatic toxicity.

However, a compound causing an increase in the bile acid toxicity potency is characterized as having a potential to cause cholestatic hepatic toxicity. The cholestatic hepatic toxicity can be caused by inhibition of bile acid efflux, wherein the compound is characterized as a bile acid efflux inhibitor, and/or the cholestatic hepatic toxicity is caused by antagonism of farnesoid X receptor (FXR), wherein the compound is characterized as a FXR antagonist, and/or a combination of both. In some aspects, inhibition of bile acid efflux comprises inhibition of a bile salt export protein (BSEP).

Still yet, a compound causing a decrease in the bile acid toxicity potency is characterized as having a potential to cause cholestasis resulting in systemic toxicity. The cholestasis can result in systemic toxicity is caused by inhibition of bile acid uptake.

In some aspects, determining the potential of the compound to cause systemic or hepatic toxicity comprises characterizing the compound as a bile acid efflux inhibitor, an FXR antagonist, an inhibitor of bile acid uptake, or none of the above. In some embodiments, the compound can be a drug candidate, wherein the drug candidate is characterized as having a low probability of causing systemic and/or hepatic toxicity, or a high probability of causing systemic and/or hepatic toxicity.

In some embodiments, bile acid transport comprises bile acid uptake, basolateral excretion and/or canalicular excretion. The range of concentrations of the bile acid can comprise intracellular concentrations mimicking *in vivo* intracellular fasting and/or postprandial concentrations. The range of concentrations of the bile acid can comprise a bile acid intracellular concentration sufficient to activate an FXR feedback mechanism.

In some aspects, determination of a toxicity profile of the bile acid can comprise measuring a hepatotoxic response using a cytotoxicity assay. Such a cytotoxicity assay can be, but is not limited to, an enzyme leakage assay, ATP, APOTOX Glo and a combination thereof. The enzyme leakage assay can comprise, for example, ALT, AST and LDH.

In some embodiments, methods of screening a compound for its potential to cause systemic and/or hepatic toxicity can further comprise exposing the HCS to a plurality of bile acids to determine toxicity profiles for the plurality of bile acids, wherein the bile acid toxicity profiles comprise a plurality of toxicity potencies. The plurality of toxicity potencies can be measured in HCS in the absence and presence of the compound to predict a hepatotoxic potential of the compound.

In some embodiments, methods of screening a compound for its potenital to cause systemic and/or hepatic toxicity can utilize an HCS including a 2-dimensional culture or 3-dimensional culture, utilizing primary hepatocytes or other hepatic cell systems. The 2-dimensional culture can comprise a sandwich culture of hepatocytes (SCH), wherein the SCH comprises human hepatocytes. The 3-dimensional culture can comprise a 3D scaffold-based culture or spheroidal culture. Other suitable hepatic cell systems include, but are not limited to, HepaRG, Huh7, co-cultured systems, stem-cell derived hepatocytes and combinations thereof.

In some embodiments the compound can be exposed to the HCS across a range of concentrations. The bile acid or bile acids is/are GCA, GCDCA, GDCA, DCA, CA, CDCA, TCA, TCDCA, LCA, GLCA, TLCA, or any combination thereof. Moreover, in some aspects one or more free fatty acids and/or a predetermined glucose concentration can be employed in the HCS.

Provided herein are *in vitro* systems for predicting *in vivo* hepatotoxic potential of a compound, comprising *in vitro* cultured HCS comprising a capacity for bile acid synthesis, bile acid transport and/or bile acid regulation, one or more bile acids with an established toxicity potency within the HCS, and an assay for determining the hepatotoxicity of a compound when exposed to the HCS in the presence of the one or more bile acids.

The *in vitro* cultured HCS can comprise an integrated hepatic cell system with bile acid synthesis, transport, and bile acid homeostasis feedback mechanisms. The *in vitro* cultured HCS can comprise a 2-dimensional culture or 3-dimensional culture, utilizing primary hepatocytes or other hepatic cell systems. The *in vitro* cultured HCS can comprise a sandwich culture of hepatocytes (SCH), wherein the SCH comprises human hepatocytes. The *in vitro* cultured HCS can comprise a 3D scaffold-based culture or spheroidal culture. The *in vitro* cultured HCS can comprise HepaRG, Huh7, co-cultured systems, stem-cell derived hepatocytes and combinations thereof.

In some aspects, in these systems the bile acid or bile acids is/are GCA, GCDCA, GDCA, DCA, CA, CDCA, TCA, TCDCA, LCA, GLCA, TLCA, or any combination thereof.

The established toxicity potency of the one or more bile acids can comprise a toxicity profile of the one or more bile acids based on a hepatotoxic response using a cytotoxicity assay. Such cytotoxicity assays can comprise, but are not limited to, an enzyme leakage assay, ATP, APOTOX Glo and a combination thereof. The enzyme leakage assay can be, for example, ALT, AST and LDH.

In some aspects, such an *in vitro* system can be configured to characterize a compound causing no change in the bile acid toxicity potencies as a compound not likely to cause systemic or hepatic toxicity. In some aspects, such an *in vitro* system can be configured to characterize a compound causing an increase in the bile acid toxicity potencies as a compound having a potential to cause cholestatic hepatic toxicity. In some aspects, such an *in vitro* system can be configured to characterize a compound causing a decrease in the bile acid toxicity potencies as a compound having a potential to cause cholestasis resulting in systemic toxicity.

In some embodiments, provided herein are methods of screening a compound for its potential to cause systemic and/or hepatic toxicity. In some embodiments, the methods comprise providing a compound to be screened, establishing a HCS comprising a capacity for bile acid synthesis, bile acid transport and/or bile acid regulation, exposing the HCS to a range of concentrations of a bile acid in the presence of the compound to be screened and determining a toxicity profile of the bile acid in the presence of the compound to be screened, wherein the toxicity profile of the bile acid is known across the range of concentrations, wherein the toxicity profile of the bile acid comprises a toxicity potency of the bile acid, and comparing the toxicity potency of the bile acid in the presence and absence of the compound to determine the susceptibility of the compound to cause systemic and/or hepatic toxicity. In such methods, the compound can be exposed to the HCS across a range of concentrations.

In some aspects, the bile acid can comprise a combination of bile acids, wherein the toxicity profile of the combination of bile acids is known, wherein the toxicity profile comprises a toxicity potency. The combination of bile acids can comprise a plurality of bile acids provided in predetermined ratios. The combination of bile acids can comprise a plurality of bile acids at concentrations and ratios configured to mimic *in vivo* concentrations and ratios of the plurality of bile acids.

Determining the potential of the compound to cause systemic or hepatic toxicity can in some aspects further comprise identifying no change in the bile acid toxicity potency in the presence of the compound as compared to the absence of the compound, identifying an increase in the bile acid toxicity in the presence of the compound as compared to the absence of the compound, or identifying a decrease in the bile acid toxicity in the presence of the compound as compared to the absence of the compound.

A compound causing no change in the bile acid toxicity potency can be characterized as not causing systemic or hepatic toxicity. Alternatively, a compound causing an increase in the bile acid toxicity potency can be characterized as having a potential to cause cholestatic hepatotoxicity. The cholestatic hepatotoxicity can be caused by inhibition of bile acid efflux, wherein the compound is characterized as a bile acid efflux inhibitor, and/or the cholestatic hepatic toxicity is caused by antagonism of FXR, wherein the compound is characterized as a FXR antagonist, and/or a combination of both. The bile acid efflux inhibition can comprise inhibition of a bile salt export protein (BSEP).

Still yet, a compound causing a decrease in the bile acid toxicity potency can be characterized as having a potential to cause cholestasis resulting in systemic toxicity. The cholestasis resulting in systemic toxicity can be caused by inhibition of bile acid uptake.

In some aspects, determining the potential of the compound to cause systemic or hepatic toxicity comprises characterizing the compound as a bile acid efflux inhibitor, an FXR antagonist, an inhibitor of bile acid uptake, or none of the above. The compound can be a drug candidate, wherein the drug candidate is characterized as having a low probability of causing systemic and/or hepatic toxicity, or a high probability of causing systemic and/or hepatic toxicity.

Bile acid transport can comprise bile acid uptake, basolateral excretion and/or canalicular excretion. The range of concentrations of the bile acid can comprise intracellular concentrations mimicking *in vivo* intracellular fasting and/or postprandial concentrations. The range of concentrations of the bile acid can comprise a bile acid intracellular concentration sufficient to activate an FXR feedback mechanism. In some aspects, the bile acid or bile acids can be, but are not limited to, GCA, GCDCA, GDCA, DCA, CA, CDCA, TCA, TCDCA, LCA, GLCA, TLCA, or any combination thereof.

In some embodiments, determination of a toxicity profile of the bile acid can comprise measuring a hepatotoxic response using a cytotoxicity assay. The cytotoxicity assay can be, for example, an enzyme leakage assay, ATP, APOTOX Glo and a combination thereof. The enzyme leakage assay can include, for example, of ALT, AST and LDH.

In such methods, the HCS can comprise a 2-dimensional culture or 3-dimensional culture, utilizing primary hepatocytes or other hepatic cell systems. The 2-dimensional culture can comprise a sandwich culture of hepatocytes (SCH), wherein the SCH comprises human hepatocytes. Alternatively, the 3-dimensional culture can comprise a 3D scaffold-based culture or spheroidal culture. Other hepatic cell systems can comprise HepaRG, Huh7, co-cultured systems, stem-cell derived hepatocytes and combinations thereof. Finally, in such methods one or more free fatty acids and/or a predetermined glucose concentration can be employed in the HCS.

### EXAMPLES

The following examples are included to further illustrate various embodiments of the presently disclosed subject matter. However, those of ordinary skill in the art should, in light of the present disclosure, appreciate that many changes can be made in the specific embodiments which are disclosed and still obtain a like or similar result without departing from the scope of the presently disclosed subject matter.

### Example 1

### Bile Acid Homeostasis

As demonstrated herein, drug induced cholestatic hepatotoxicity is the result of impaired bile flow of bile acids via inhibition of BSEP by the NCE and impairment of the hepatocyte bile acid homeostasis adaptive response. One possible mechanism of action causing drug induced cholestatic hepatotoxicity can comprise a cholestatic hepatotoxicant inhibiting BSEP function (initial insult) and 1) preventing (antagonize) Farnesoid X Receptor (FXR) from sensing the rising intracellular concentrations of bile acids; or 2) inhibiting all bile acid efflux pathways (basolateral and canalicular); or 3) a combination of both.

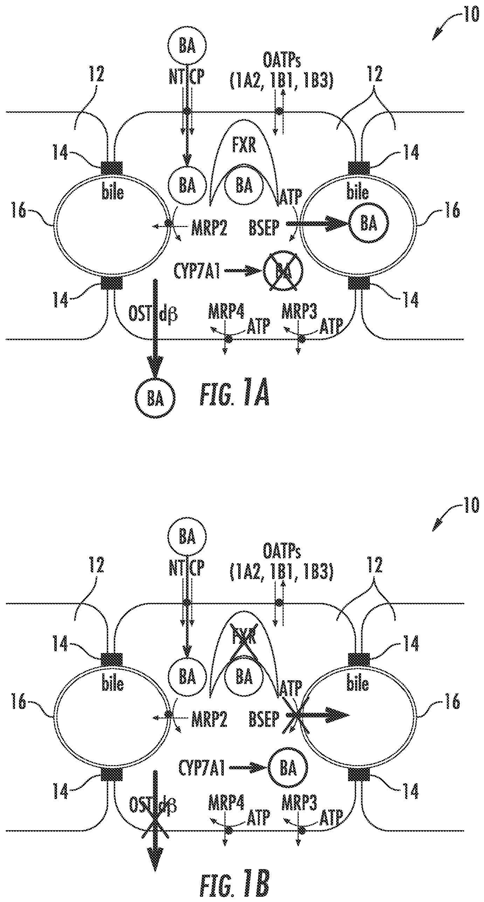

Figures 1A through 6 are schematic illustrations of a hepatic cell system **10** (*in vivo* or *in vitro*) comprising hepatocytes **12** with tight junctions **14** and one or more bile canaliculi **16** therebetween. Components, enzymes and transporters within the cell systems include: bile acid **BA**; adenosine triphosphate **ATP**; Farnesoid X Receptor **FXR**; Bile Salt Export Protein **BSEP**; multidrug resistance-associated protein 2 **MRP2**;multidrug resistance-associated protein 3 **MRP3**; multidrug resistance-associated protein 4 **MRP4**; normal bile acid uptake **NTCP**; basolateral efflux transporter **OST**; bile acid synthetic enzyme **Cyp7A1,** as discussed and defined herein.

Figure 1A illustrates normal bile acid homeostatic pathways *in vivo,* whereas Figure 1B illustrates compromised bile acid homeostasis potentially leading to drug induced cholestatic hepatotoxicity. In Figure 1B, one or more of **FXR**, **BSEP** and/or **OST** are blocked or inhibited causing an interference in bile acid homeostasis and transport.

Thus, in some embodiments, the screening assays, methods and systems provided herein include components of the following concepts:
- BSEP inhibition;
- Bile acid homeostasis (FXR) feedback mechanism;
- Understanding the need to utilize an integrated hepatic cell system that maintains bile acid transport and bile acid homeostasis feedback mechanism; SCHH system fulfills first two items above;
- Realization that SCHH system requires additional bile acids to behave more like *in vivo* (e.g. fasting and postprandial); system employs BA dose response to model fasting and postprandial status in the presence and absence of NCE;
- Identifying physiologically relevant bile acid or bile acid pool that elicits toxic response (i.e. DCA); and/or
- Linking cholestasis to toxicity in an *in vitro* assay for the first time.

### Example 2

### Bile Acid Homeostasis Feedback Mechanisms

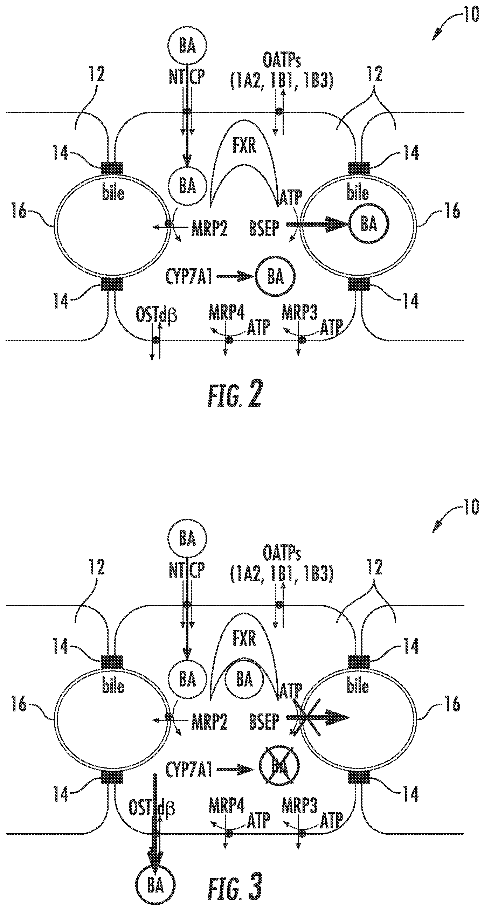

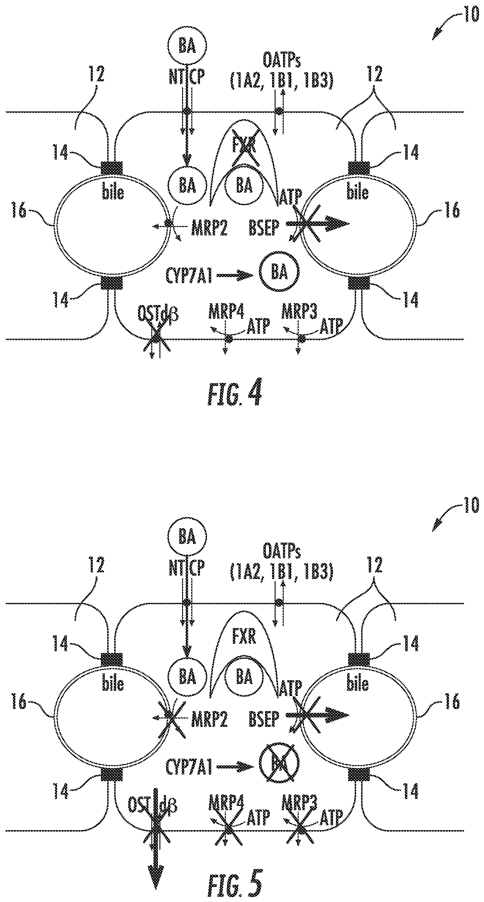

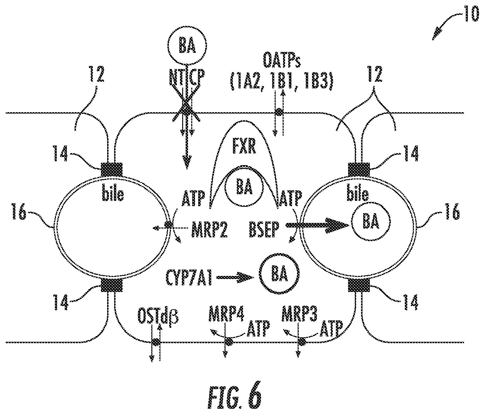

Figure 2 illustrates normal bile acid uptake (NTCP), synthesis (CYP7A1) and export (BSEP) *in vivo.* Figure 3 illustrates the effects of a NCE acting as a BSEP inhibitor initiating the bile acid homeostasis feedback mechanism (e.g. activation of FXR) resulting in the induction of the compensatory mechanism (e.g. basolateral efflux transporter OSTα/β) and lowering of the intracellular concentrations of bile acids. Initiating the bile acid feedback mechanism prevents bile acid hepatotoxicity (aka cholestatic hepatotoxicity). Figure 4 illustrates the effects of a NCE acting as a BSEP inhibitor and FXR antagonist preventing the activation of the bile acid homeostasis feedback mechanism resulting in increased bile acid intracellular concentrations leading to bile acid hepatotoxicity (aka cholestatic hepatotoxicity). Figure 5 illustrates the effects of a NCE inhibiting multiple bile acid efflux routes, resulting in hepatotoxicity. Bile acid feedback mechanism remains intact; however, the compensatory mechanism (e.g. OSTα/β basolateral efflux) and other bile acid efflux mechanisms are inhibited leading to increased bile acid intracellular concentrations and hepatotoxicity, i.e. cholestatic hepatotoxicity. Figure 6 illustrates the effects of a NCE inhibiting bile acid uptake, resulting in reducing the amount of bile acids presented to the hepatocyte leading to systemic cholestasis.

### Example 3

Cholestasis Hepatotoxicity Screening Assay Development Assays, methods and systems are provided herein to identify NCE that have the potential to interfere with the hepatocyte's bile acid handling capacity. In some embodiments, such assays, methods and systems are configured to link cholestasis to hepatotoxicity based on toxicity assays (e.g. ATP/LDH/Caspase 3/7/8), and/or by monitoring LDH which is relatable to clinical measurements (AST/ALT) typically used to monitor liver function. In some embodiments, such assays, methods and systems are configured to categorize an NCE as being cholestatic, having no effect, or causing cholestatic hepatotoxicity.

Potential Outcomes (BA in the presence of NCE) can in some embodiments be expressed as illustrated in Figures 7 and 8. In Figures 7 and 8 the dotted, dashed and solid lines are indicative of the following:
- Dotted
   - BA dose response to determine BA toxicity potency (TC₅₀) in the absence of NCE
      - Calibration of system ID normal response
      - Demonstrates how hepatocytes respond over a large concentration range of BA
   - No change in BA toxicity potency in presence of NCE = No predicted effect
- Dashed
   - Increase in BA toxicity potency in the presence of NCE = potential to cause cholestatic hepatotoxicity
   - BA Efflux inhibitor, FXR antagonist, or both
- Solid
   - Decrease in BA toxicity potency in the presence of NCE equals potential to cause cholestasis may result in systemic toxicity
   - BA uptake inhibitor

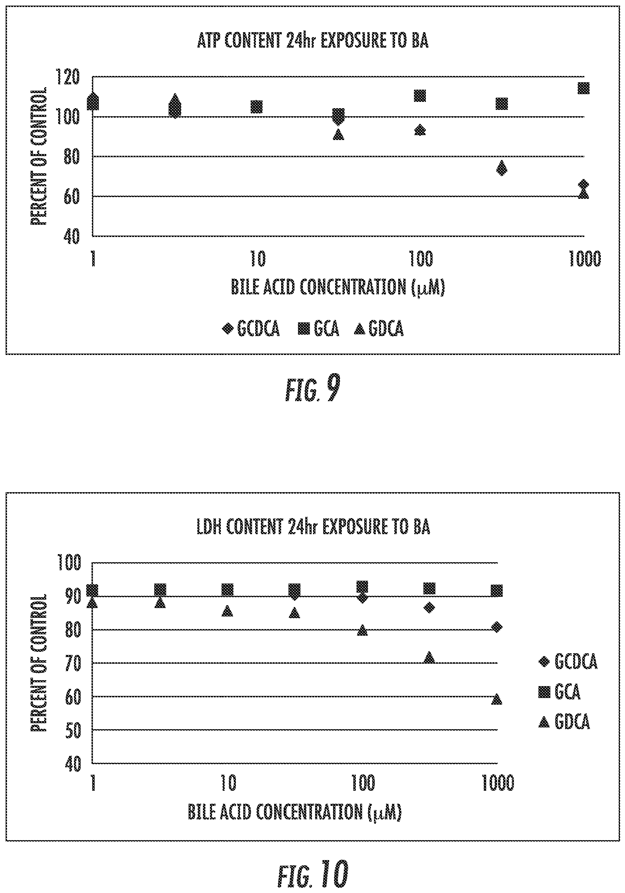

Bile acids GCA, GCDCA and GDCA were evaluated at increasing concentrations in SCHH over 24 hours. ATP (Figure 9) and LDH (Figure 10) content were measured. The results of these evaluations, as shown in Figures 9 and 10, demonstrate that SCHH is quite resistant to BA toxicity within 24 hr exposure. More particularly, GCA was not cytotoxic, GCDCA equaled GDCA in the ATP assay, and GDCA was greater than GCDCA in the LDH assay.

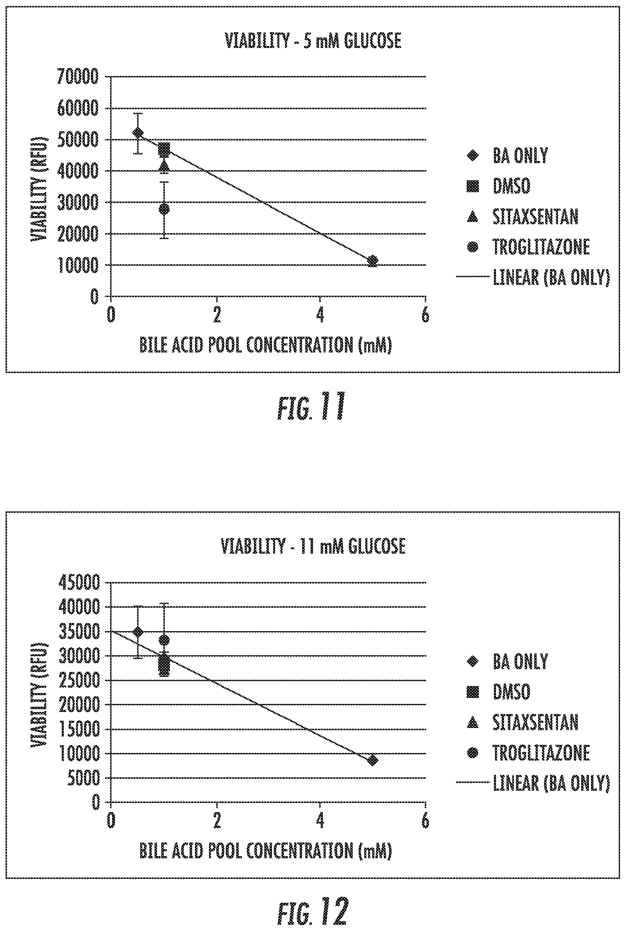

Glucose concentrations were evaluated to examine the impact of glucose concentrations on bile acid pool toxicity profiles, i.e., shifts, in the absence and presence of cholestatic agents, e.g. troglitazone and sitaxsentan, in SCHH. Utlizing similar methodology from the previous sections, hepatotoxicity was evaluated with ApoTox-Glo^{™} Triplex Assay. The results are shown in Figures 11 and 12.

Low glucose conditions were needed to increase the SCHH system susceptibility to the troglitazone effect on cell viability and bile acid potency relationship (Figures 11-12). Low glucose conditions improved dynamic range of cell viability assay presumably by improving the hepatoyctes dependency on mitochondrial function leading to increased ATP output and mitochondrial function. The addition of sitaxsentan did not alter the hepatotoxicity of the bile acid pool.

### Example 4

### Cholestasis Hepatotoxicity Screening Assay Feasibility

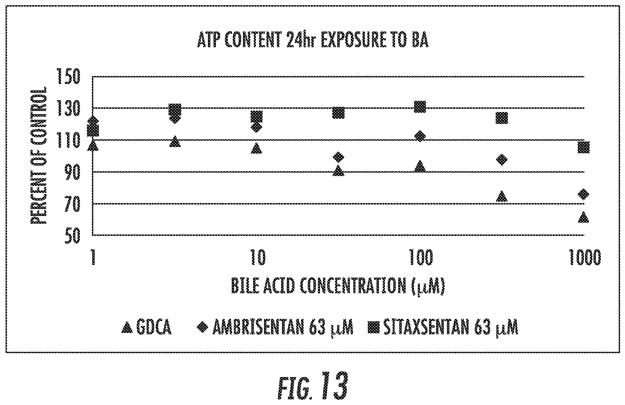

A feasibility study was also conducted to determine the ability of the disclosed assays, methods and systems to distinguish Ambrisentan from Sitaxsentan, the results of which are below and in Figure 13. Characteristics of Ambrisentan from Sitaxsentan are listed below:
- Sitaxsentan (2-200 uM)
   - Decreased BA toxicity potency
   - *In vitro* assays indicated potent/efficacious NTCP inhibitor
   - *In vitro* assay showed no BSEP effect
   - Cmax (the maximum (or peak) concentration achieved after administrated) = 23.5 uM
   - Toxicity in clinic after 5 months; liver likely not primary site of toxicity
      - No toxicity @ 3 months
- Ambrisentan (2-200 uM)
   - No change in BA toxicity potency
   - Cmax = 3 uM
   - Well tolerated; no toxicity
   - *In vitro* assays showed no NTCP/BSEP effect

The effects of individual bile acids and a mixture of bile acids (bile acid pool) were evaluated in sandwich-cultured human hepatocytes following a 24 hour exposure time in the presence of 5 mM glucose, for their potential to alter the hepatotoxicity of troglitazone (100 µM) and sitaxsenten (60 µM). The effects of the test compounds were evaluated in the presence of the individual bile acids and in the presence of a bile acid pool. The effects were compared to bile acid treatment alone. DMSO treatment alone was used as a comparator for hepatotoxicity. Hepatotoxicity was evaluated with ApoTox-Glo^{™} Triplex Assay, which assesses viability, cytotoxicity and apoptosis.

**Table 1.**

| Composition of Bile Acid Pool | | | |
|---|---|---|---|
| Indiv. BA Conc. (mM) | For 5 mM Total: (mM) | For 1 mM Total: (mM) | For 0.5 mM Total: (mM) |
| GCDCA | 2.64 | 0.529 | 0.264 |
| GCA | 1.13 | 0.227 | 0.113 |
| DCA | 0.634 | 0.127 | 0.063 |
| GDCA | 0.588 | 0.118 | 0.059 |

A bile acid mixture was evaluated, and was composed of 4 different bile acids maintained at a physiological relevant ratio of each bile acid. Table 1. Individual bile acids were dosed using the concentration they represent in the mixture.

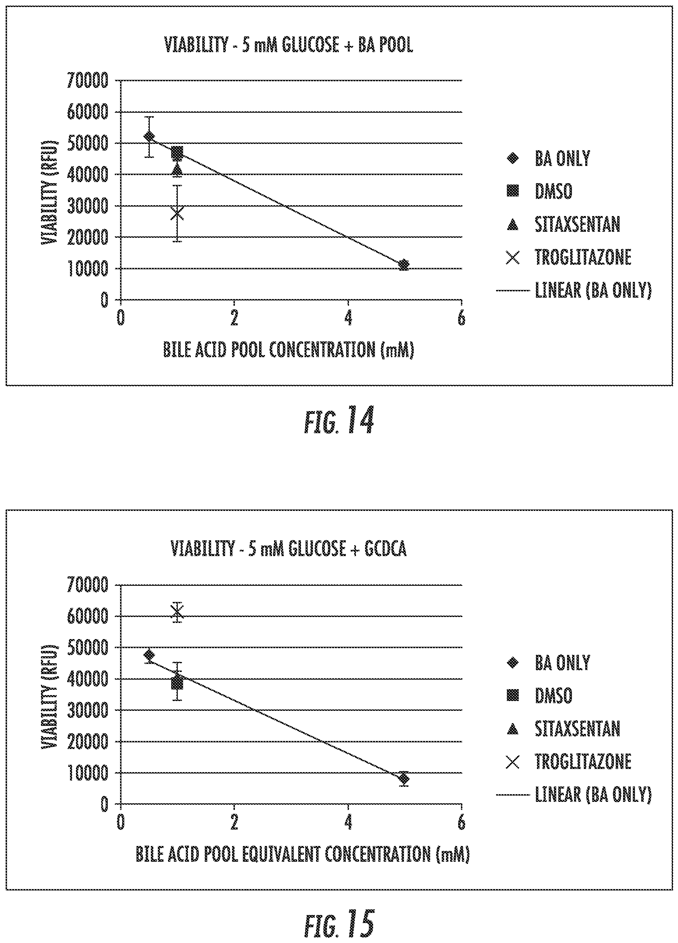

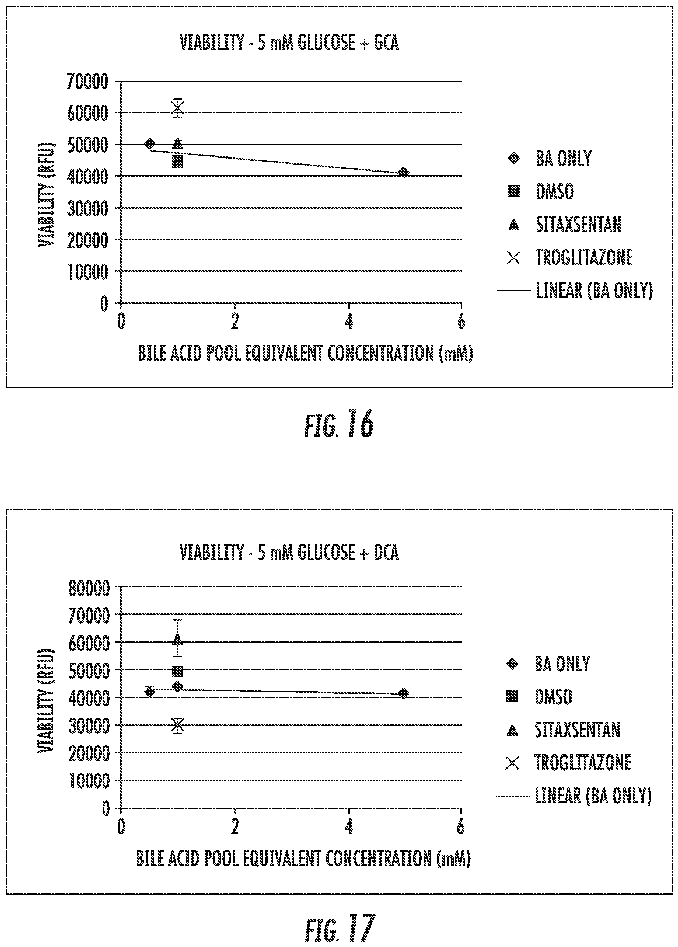

A left shift for troglitazone was observed only in the presence of the bile acid pool, indicating that less bile acids were required to decrease cell viability (Figure 14). Exposure to individual bile acids did not result in any left shift in the cell viability (Figures 15-18).

These data suggest that a bile acid pool is, in some embodiments, a necessary component to allow the system to differentiate compounds effects on bile acid homeostasis. Troglitazone interferes with the ability of the hepatocyte to respond to increased intracellular concentrations of bile acids, and thereby increasing the hepatotoxicity of the bile acids.

### Example 5

### Effects of Free Fatty Acids on the Hepatotoxicity of Test Compounds

Utilizing similar methodology from the previous section, the effect of troglitazone (100 µM) and sitaxsenten (60 µM) on the hepatotoxicity of a bile acid pool was evaluated in the presence of two free fatty acid ratios (2:1, and 0:3 Oleate:Palmitate). A treatment group without free fatty acids was used as a control. Hepatotoxicity was evaluated with ApoTox-Glo^{™} Triplex Assay which assessed viability, cytotoxicity and apoptosis.

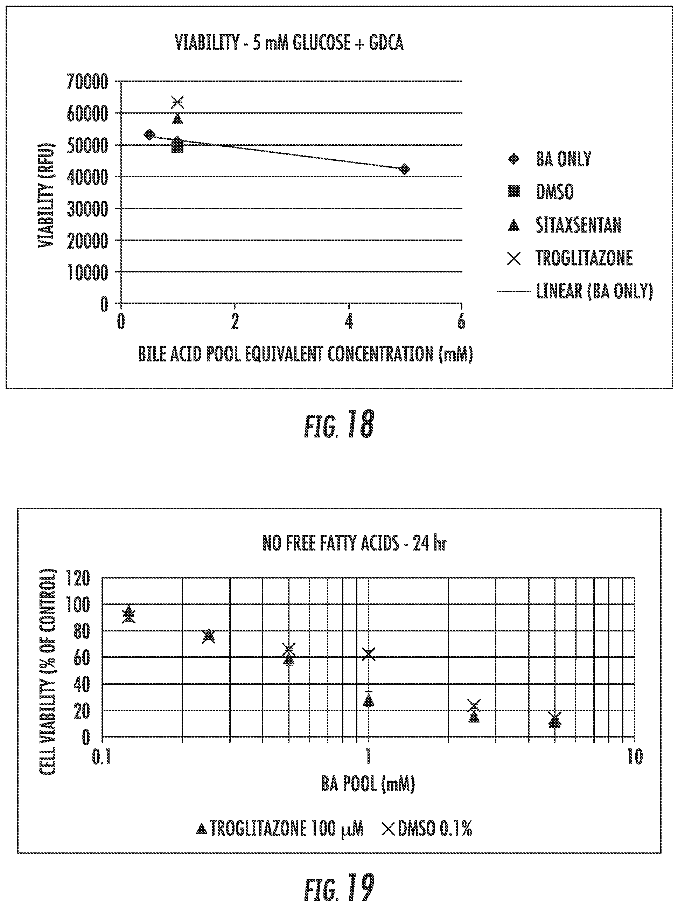

In the absence of free fatty acids the hepatotoxicity of the bile acids was increased in the presence of troglitazone (Figure 19). The TC₅₀ (concentration of bile acids that was toxic to 50% of the hepatocytes) was decreased to 44% of control from 1.48 µM to 0.65 µM (Table 2).

**Table 2.**

| | DMSO | Troglitazone |
|---|---|---|
| Slope | -26.1 | -61.3 |
| Intercept | 88.5 | 89.8 |
| TC50 | 1.48 | 0.65 |
| Shift | | 2.27 |

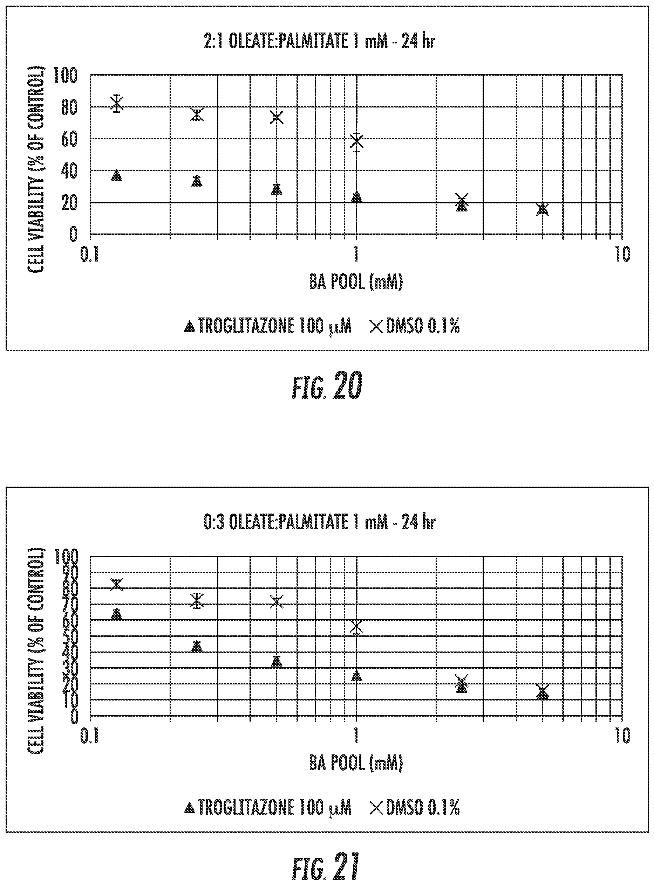

The addition of free fatty acids at both ratios of oleate:palmitate resulted in additional changes in the hepatotoxicity of the bile acid pool in the presence of troglitazone. See Figure 20. The TC₅₀ for bile acids in the presence of troglitazone and 2:1 oleate:palmitate ratio was decreased to 9.5 % of control from 1.32 µM to 0.13 µM (Table 3).

**Table 3.**

| | DMSO | Troglitazone |
|---|---|---|
| Slope | -24.2 | |
| Intercept | 81.9 | |
| TC50 | 1.32 | 0.13 |
| Shift | | 10.5 |

A similar effect was observed in the presence of a 0:3 oleate:palmitate ratio (Figure 21). The TC₅₀ for bile acids in the presence of troglitazone and 0:3 oleate:palmitate ratio was decreased to 17 % of control from 1.28 µM to 0.22 µM (Table 4).

**Table 4.**

| | DMSO | Troglitazone |
|---|---|---|
| Slope | -22.6 | -160.5 |
| Intercept | 79.0 | 84.8 |
| TC50 | 1.28 | 0.22 |
| Shift | | 5.91 |

Addition of free fatty acids alone at either ratio of oleate:palmitate minimally affected the hepatotoxicity profile of pooled bile acids. The TC₅₀ of bile acids in the DMSO control was minimally reduced by 89% and 86% of Control, respectively, with the addition of either the 2:1 or 0:3 oleate:palmitate ratio of free fatty acids. See Table 5 below.

**Table 5.**

| | DMSO | 2:1 | 0:3 |
|---|---|---|---|
| Slope | -26.1 | -24.2 | -21.0 |
| Intercept | 88.5 | 81.9 | 71.9 |
| TC50 | 1.48 | 1.32 | 1.28 |
| % of control | 100% | 89.1% | 86.4% |

This unanticipated effect of free fatty acids on the hepatotoxicity of bile acids in the presence of troglitazone, suggests that the addition of free fatty acids may be a factor in the evaluation of test compounds for their potential to alter bile acid disposition and to predict hepatotoxicity.

### Example 6

### Application of Cholestasis Hepatotoxicity Screening Assay

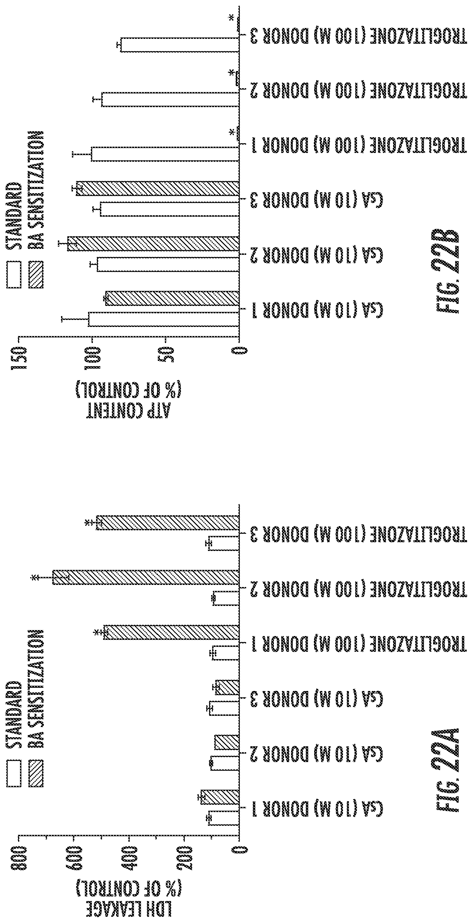

As discussed above, hepatocytes become susceptible to bile acid toxicity (e.g. cholestatic hepatotoxicity) when drug exposure inhibits bile acid efflux routes (e.g. BSEP and/or MRP3/4) and 1) prevents (antagonize) FXR from sensing the rising intracellular concentrations of bile acids; or 2) inhibits bile acid efflux through OSTα/β; or 3) a combination of both. To further illustrate this discovery, SCHH were exposed to either a BSEP inhibitor (e.g. cyclosporine A (CsA; Ansede et al., 2010) or a compound with both BSEP inhibitor and FXR antagonist characteristics (e.g. troglitazone; Marion et al., 2007 and Kaimal et al. 2009) for 24 hours using a low glucose (about 2 mM to about 10 mM, or about 5 mM) standard culture medium or sensitization medium containing low glucose (about 2 mM to about 10 mM, or about 5 mM), or in some embodiments other suitable energy sources, e.g. galactose, free fatty acids (about 100 µM to about 2 mM, or about 250 µM to about 1.5 mM, or about 500 µM to about 1 mM, or about 1 mM), and a bile acid pool (about 5 µM to about 1 mM, or about 50 µM to about 500 µM, or about 100 µM to about 250 µM, or about 250 µM). Briefly, hepatocytes were established in a sandwich-cultured configuration for four days. On day four of culture, SCHH were exposed to CsA (10 µM; 13X Cmax) or troglitazone (100 µM; 16X Cmax) for 24 hours cultured in standard culture medium or sensitization medium. Concentrations of CsA and troglitazone were based on Cmax values reported in Dawson et al. 2012. Drug exposures ranged from about 8X to about 16X Cmax to account for concentrating effect that can occur in the portal vein following oral administration of test compounds. Following 24 hours, LDH leakage and ATP content was evaluated utilizing commercially available assays. No marked increases of LDH secretion and no significant decreases in ATP content were observed in any of the three SCHH donors treated with either CsA (10 µM; 13X Cmax) or troglitazone (100 µM; 16X Cmax) under standard culture conditions (Figures 22A and 22B). However under sensitization culture conditions, troglitazone and not CsA exposure significantly increased LDH secretion ≥ 490% of control (Tukey's; p-value < 0.05) and reduced ATP content to ≤ 0.9% of control (Tukey's; p-value < 0.05) across all three SCHH donors (Figures 22A and 22B).

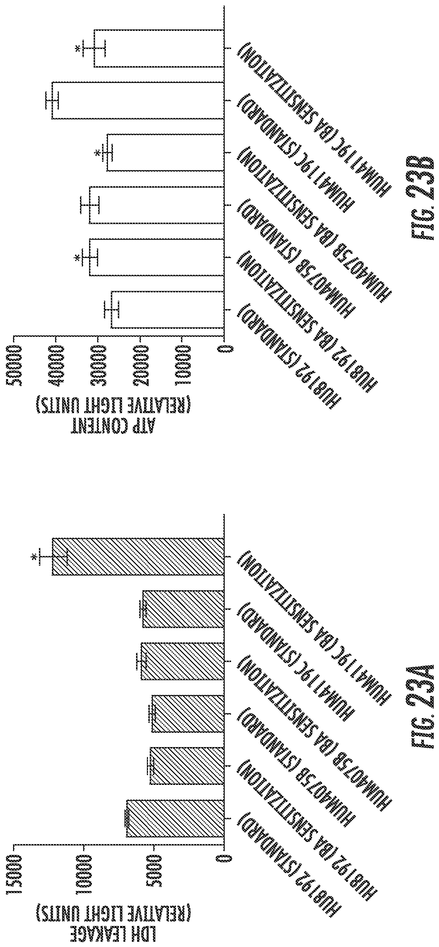

The sensitization medium included free fatty acids and a physiological mixture of primary and secondary bile acids, described above (GCDCA, GCA, DCA, and GDCA) at an adequate concentration (250 µM) that minimized toxicity but challenged the hepatocytes bile acid homeostasis mechanism. Following 24 hours of exposure to sensitization media, LDH secretion was not significantly (Tukey's; p-value < 0.05) increased in two out of the three SCHH preparations evaluated (Figure 23A). In the same donor, ATP content was decreased ≤ 24.8% in sensitization media (Figure 23B).

These results demonstrated that sensitization media was well-tolerated across multiple donors.

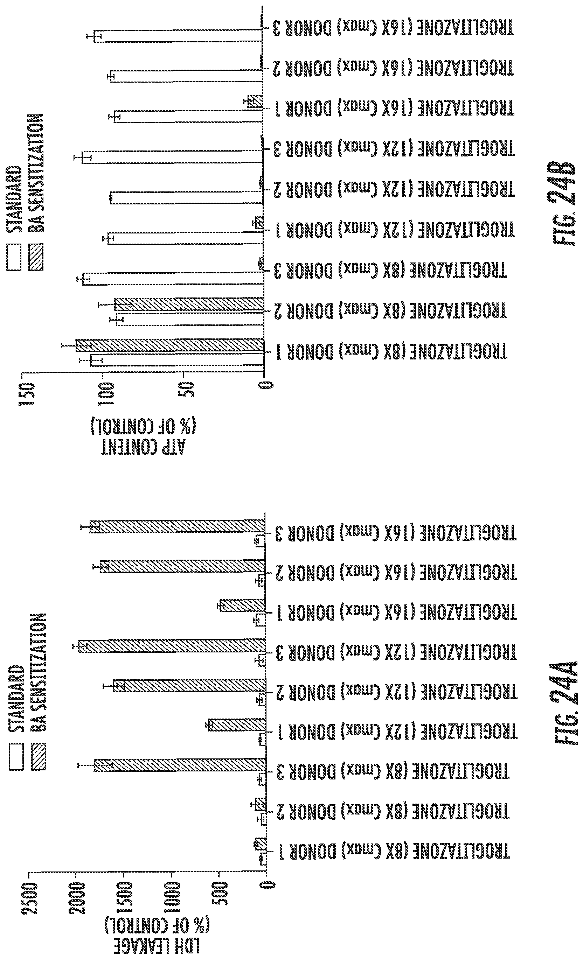

Hepatocytes under standard culture conditions do not have a sufficient concentration of bile acids or proper mixture to induce toxicity in the presence of a cholestatic hepatotoxicant, troglitazone. This was demonstrated by the lack of an effect on LDH and ATP under standard culture conditions across all three donors examined when treated with troglitazone (Figures 24A and 24B). Hepatocytes require a bile acid load to fully evaluate the function of the bile acid homeostatic mechanisms. More than 95% of secreted bile acids following a meal are reabsorbed *in vivo* from the ileum into the portal vein (Chiang, 2009). Thus, the *in vivo* bile acid portal vein concentrations are dynamic and have been reported to increase ≥3-fold between fasting and postprandial states (Angelin B et al, 1982) represented by standard and sensitization culture medium, respectively.

The primary human toxicities associated with CsA therapy are nephrotoxicity and neurotoxicity, not liver toxicity, and has a relatively low incidence of occurrence when compared to the usage (LiverTox Database, Magnasco et al., 2008). In contrast, the clinical evidence regarding troglitazone estimates that the incidence of liver injury can be as high as 1:1000 patients and clearly indicates that troglitazone causes liver injury (LiverTox Database). The sharp contrast in clinical liver injury incidence between these two potent BSEP inhibitors suggested that in addition to BSEP inhibition another process is likely involved, such as interference (e.g. FXR antagonism) of the bile acid homeostasis mechanism. The clinical liver injury incidence and *in vitro* assay results of CsA and troglitazone were in greet agreement suggesting that the cholestatic hepatotoxicity assay is capable of distinguishing between a non-DILI agent, CsA, and a cholestatic DILI agent, troglitazone.

Titration of troglitazone, a thiazolidinedione class compound, of 50 µM (8X Cmax), 75 µM (12X Cmax), and 100 µM (16X Cmax) across three different SCHH preparations established dose-dependency of the toxicity manifesting in only the sensitization media (Figures 24A and 24B). Marked LDH leakage of ≥590% of control and loss of ATP content of ≥ 95% of control was observed across all three donors treated with troglitazone at concentrations between 50 to 75 µM (8X-12X Cmax; Figure 24B). These results demonstrated that the hepatotoxicity pathway was not idiosyncratic, but conserved across donors with the toxicity being dose-dependent. No marked increases of LDH leakage or loss of ATP was observed in SCHH exposed to troglitazone under standard media conditions at any concentration evaluated. These results demonstrated that SCHH require certain culture conditions (e.g. sensitization medium) to identify cholestatic DILI agents. These results suggested that troglitazone disrupts the bile acid homeostasis mechanism resulting in bile acid or cholestatic hepatotoxicity when conditions require hepatocytes to process a large amount of bile acids (e.g. sensitization conditions).

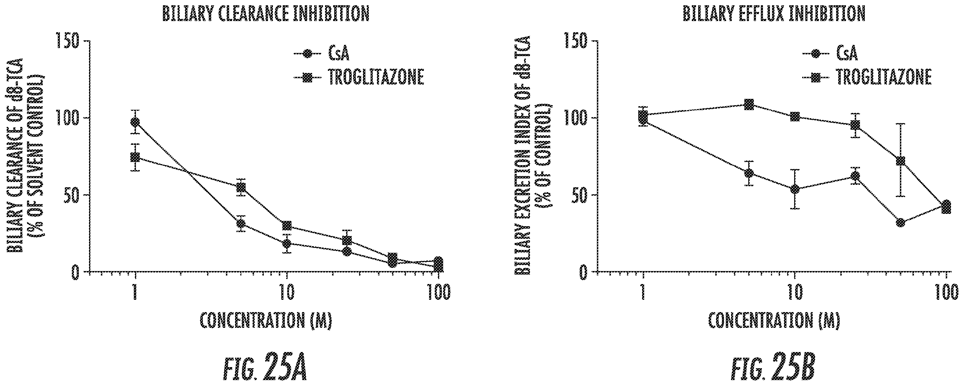

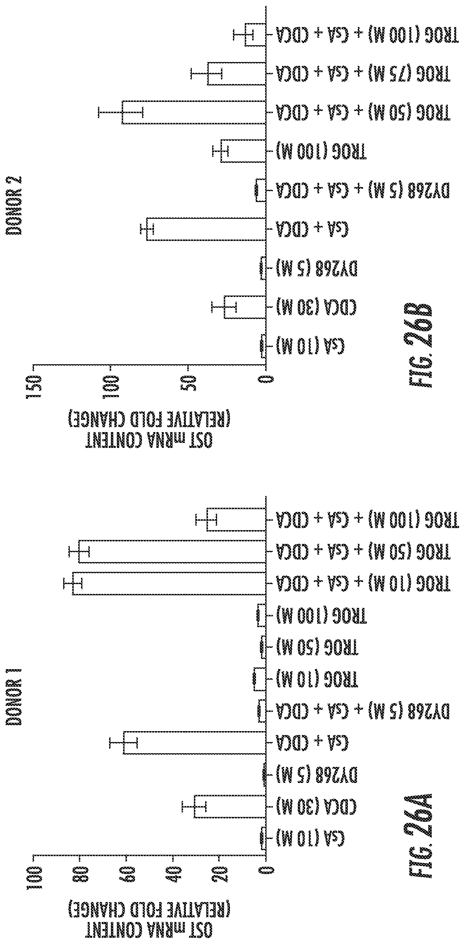

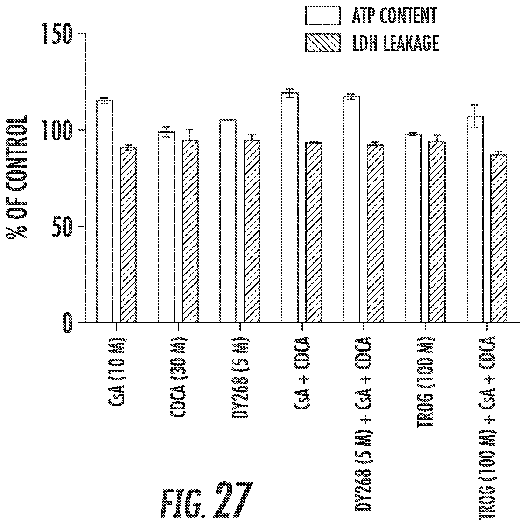

This conclusion was further supported by inhibition of biliary clearance of d8-TCA (Figure 25) and FXR antagonism (Figure 26) observed in SCHH cultures in the presence of troglitazone. Biliary clearance of the model bile acid, d8-TCA, was markedly reduced in a dose-dependent manner in SCHH treated with troglitazone (Figure 25A). Biliary excretion of d8-TCA (e.g. biliary excretion index) was also significantly reduced in a dose-dependent manner in SCHH exposed to troglitazone (Figure 25B). These results suggested that bile acid excretion across the canalicular domain was reduced in the presence of troglitazone consistent with previous reports (Marion et al., 2007). Troglitazone (75-100 µM) exposure also prevented the synergistic induction response of OSTβ mRNA content observed in SCHH following co-treatment with CDCA and CsA (Figure 26A and 26B). A similar effect was observed in SCHH treated with a combination of CDCA, CsA, and DY268, a novel and potent FXR antagonist (Yu et al., 2014). Alternatively, the reduction of OSTβ mRNA content may be explained by cytotoxicity under the conditions evaluated. However, no significant decreases of ATP content or increases of LDH leakage were observed under the conditions examined suggesting that the reduction of OSTβ mRNA content was due to antagonism of FXR activation (Figure 27).

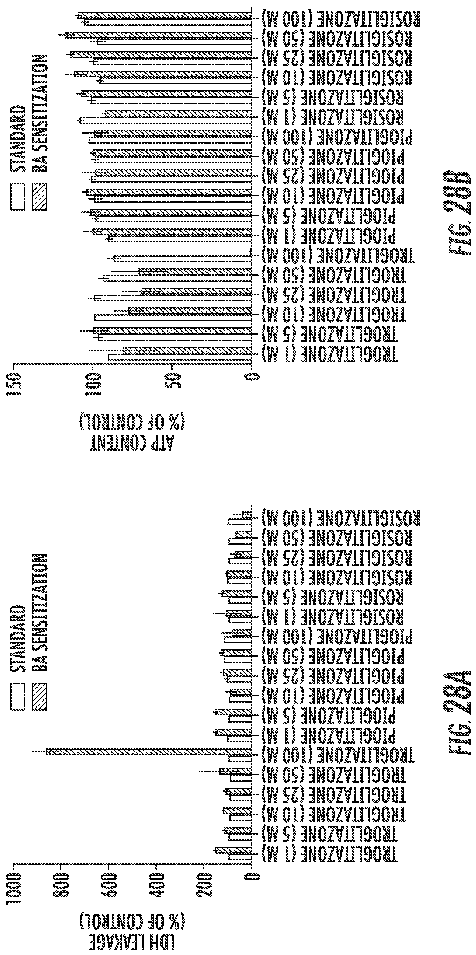

Other thiazolidinediones including pioglitazone and rosiglitazone were utilized as a preliminary evaluation of the cholestatic hepatotoxicity screen prediction accuracy. Troglitazone, a thiazolidinediones and well-established DILI agent, has clinical liver injury incidence of 1:1000 patients and was withdrawn from market due to liver injury (LiverTox Database). In contrast, fewer than 12 liver injury cases have ever been reported for either of pioglitazone and rosiglitazone despite extensive use (LiverTox Database). In large clinical trials, ALT elevations ≥ 3X upper limit normal were not different from placebo recipients for either pioglitazone or rosiglitazone suggesting low potential for liver injury for either of these thiazolidinediones. All three thiazolidinediones were evaluated in the cholestasis hepatotoxicity screening assay across a wide range of concentrations (1, 5, 10, 25, 50, 100 µM) encompassing the Cmax for each troglitazone (Cmax: 6.4 µM; Dawson et al. 2012), pioglitazone (Cmax: 2.9 µM; Dawson et al. 2012), and rosiglitazone (Cmax: 1.0 µM; Dawson et al. 2012). A marked increase of LDH leakage (863% of control) and marked loss of ATP content (>99% of control) was observed in SCHH treated with troglitazone (100 µM) under sensitization culture conditions only (Figure 28A and 28B) consistent with previous results. No noticeable increases of LDH leakage or decreases of ATP content were observed in SCHH treated with pioglitazone or rosiglitazone at any concentration under either culture condition examined (Figures 28A and 28B). These *in vitro* results were largely in agreement with clinical liver injury incidence within the thiazolidinediones class of compounds demonstrating the predictive accuracy of the disclosed cholestatic hepatotoxicity screening assay.

### REFERENCES

Ansede JH, Smith WR, Perry CH, St. Claire III RL, and Brouwer KR (2010) An in vitro assay to assess transporter-based cholestatic hepatotoxicity using sandwich-cultured rat hepatocytes. Drug Metab and Dispos 38:276-280.
Marion TL, Leslie EM and Brouwer KLR (2007) Use of sandwich-cultured hepatocytes to evaluate impaired bile acid transport as a mechanism of drug-induced hepatotoxicity. Mol Pharmaceutics 4(6): 911-918.
Kaimal R, Song X, Yan B, King R, and Deng R (2009) Differential modulation of farnesoid X receptor signaling pathway by the thiazolidinediones. J Pharmacol Exp Ther 330: 125-134.
Dawson S, Stahl S, Paul N, Barber J, and Kenna JG (2012) In vitro inhibition of the bile salt export pump correlates with risk of cholestatic drug-induced liver injury in humans. Drug Metab and Dispos 40: 130-138.
Angelin B, Bjorkhem I, Einarsson K, and Ewerth S (1982) Hepatic uptake of bile acids in man. Fasting and postprandial concentrations of individual bile acids in portal venous and systemic blood serum. J Clin Invest. 70: 724-731.
Chiang JYL (2009) Bile acids: regulation of synthesis. J Lipid Res 50: 1955-1966.
Magnasco A, Rossi A, Catarsi P, Gusmano R, Ginevri F, Perfumo F, Ghiggeri GM (2008) Cyclosporin and organ specific toxicity: clinical aspects, pharmacogenetics and perspectives. Curr Clin Pharmacol. 3(3):166-73.
Yu DD, Lin W, Forman BM, and Chen T (2014) Identification of trisubstituted-pyrazol carboxamide analogs as novel and potent antagonists of farnesoid X receptor. Bioorg Med Chem 22: 2919-2938.
Dawson S, Stahl S, Paul N, Barber J, and Kenna JG (2012) In vitro inhibition of the bile salt export pump correlates with risk of cholestatic drug-induced liver injury in humans. Drug Metab and Dispos 40: 130-138.
Hartman et al. Can J. Physiol. Pharmacol. 2010. 88:682-691.

It will be understood that various details of the presently disclosed subject matter may be changed without departing from the scope of the presently disclosed subject matter. Furthermore, the foregoing description is for the purpose of illustration only, and not for the purpose of limitation.

## Claims

1. A method of screening a compound for its potential to causing systemic and/or hepatic toxicity, the method comprising the steps of:
(a) providing a compound to be screened;
(b) establishing a hepatic cell system (HCS) comprising a capacity for bile acid synthesis, bile acid transport and/or bile acid regulation;
(c) exposing the HCS to a range of concentrations of a bile acid to determine a toxicity profile of the bile acid, wherein the bile acid toxicity profile comprises a toxicity potency;
(d) exposing the HCS to a range of concentrations of a bile acid in the presence of the compound to be screened and determining a toxicity profile of the bile acid, wherein the bile acid toxicity profile comprises a toxicity potency; and
(e) comparing the toxicity potency of the bile acid between steps (c) and (d) to determine the potential of the compound to cause systemic and/or hepatic toxicity,
wherein an increase in the toxicity potency in the step (d) as compared to step (c) indicates a potential to cause cholestatic hepatotoxicity,
wherein a decrease in the toxicity potency in step (d) as compared to step (c) indicates a potential to cause cholestasis resulting in systemic toxicity, and
wherein determining the potential of the compound to cause systemic and/or hepatic toxicity comprises (1) characterizing a compound as having a potential to cause cholestatic hepatic toxicity if the compound causes an increase in the bile acid toxicity potency; and (2)
determining whether the compound is a bile acid efflux inhibitor, a farnesoid X receptor (FXR) antagonist, or a combination thereof.

2. The method of claim 1, wherein determining the potential of the compound to cause systemic or hepatic toxicity further comprises identifying no change in the bile acid toxicity potency in the presence of the compound as compared to the absence of the compound, identifying an increase in the bile acid toxicity in the presence of the compound as compared to the absence of the compound, or identifying a decrease in the bile acid toxicity in the presence of the compound as compared to the absence of the compound.

3. The method of claim 2, wherein a compound causing nc change in the bile acid toxicity potency is characterized as not causing systemic or hepatic toxicity,

4. The method of claim 1, wherein the cholestatic hepatic toxicity is caused by inhibition of bile acid efflux and/or a bile salt export protein (BSEP), wherein the compound is characterized as a bile acid efflux inhibitor, and/or the cholestatic hepatic toxicity is caused by antagonism of farnesoid X receptor (FXR), wherein the compound is characterized as a FXR antagonist, and/or a combination of both.

5. The method of claim 2, wherein a compound causing a decrease in the bile acid toxicity potency is characterized as having a potential to cause cholestasis resulting in systemic toxicity, wherein the cholestasis resulting in systemic toxicity is caused by inhibition of bile acid uptake.

6. The method of any one of claims 1-5, wherein determining the susceptibility of the compound to cause systemic or hepatic toxicity comprises characterizing whether the compound is an inhibitor of bile acid uptake,

7. The method of any one of claims 1-6, wherein the compound is a drug candidate, wherein the drug candidate is characterized as having a low probability of causing systemic and/or hepatic toxicity, or a high probability of causing systemic and/or hepatic toxicity.

8. The method of any one of claims 1-7, wherein bile acid transport comprises bile acid uptake, basolateral excretion and/or canalicular excretion.

9. The method of any one of claims 1-8, wherein the range of concentrations of the bile acid comprises intracellular concentrations mimicking in vivo intracellular fasting and/or postprandial concentrations, and/or wherein the range of concentrations of the bile acid comprises a bile acid intracellular concentration sufficient to activate an FXR feedback mechanism.

10. The method any one of claims 1-9, wherein the determination of a toxicity profile of the bile acid comprises measuring a hepatotoxic response using a cytotoxicity assay, wherein the cytotoxicity assay is selected from the group consisting of an enzyme leakage assay, ATP, APOTOX Glo and a combination thereof, wherein the enzyme leakage assay is selected from the group consisting of ALT, AST and LDH.

11. The method of any one of claims 1-10, further comprising exposing the HCS to a plurality of bile acids to determine toxicity profiles for the plurality of bile acids, wherein the bile acid toxicity profiles comprise a plurality of toxicity potencies.

12. The method of claim 11, wherein the plurality of toxicity potencies are measured in HCS in the absence and presence of the compound to predict a hepatotoxic potential of the compound.

13. The method of any one of claims 1-12, wherein the HCS comprises a 2-dimensional culture or 3-dimensional culture, utilizing primary hepatocytes or other hepatic cell systems, wherein the 2-dimensional culture comprises a sandwich culture of hepatocytes (SCH), wherein the SCH comprises human hepatocytes, wherein the 3-dimensional culture comprises a 3D scaffold-based culture or spheroidal culture.

14. The method of any one of claims 1-13, wherein the compound is exposed to the HCS across a range of concentrations.

15. The method of any one of claims 1-14, wherein the bile acid or bile acids is/are GCA, GCDCA, GDCA, DCA, CA, CDCA, TCA, TCDCA, LCA, GLCA, TLCA, or any combination thereof, wherein one or more free fatty acids and/or a predetermined glucose concentration is employed in the HCS.

## Patentansprüche

1. Verfahren zum Screening von Verbindungen auf ihr Potential systemische und/oder hepatische Toxizität zu verursachen, umfassend folgende Schritte:
a) Bereitstellen einer zu untersuchenden Verbindung;
b) Bildung eines Leberzellsystems (HCS) mit der Fähigkeit zur Gallensäuresynthese, zum Gallensäuretransport und/oder zur Gallensäureregulierung;
c) Aussetzen des HCS einer Reihe von Konzentrationen einer Gallensäure, um ein Toxizitätsprofil der Gallensäure zu bestimmen, wobei das Gallensäure-Toxizitätsprofil eine toxische Wirkungsstärke umfasst;
d) Aussetzen des HCS einer Reihe von Konzentrationen einer Gallensäue bei Anwesenheit der zu untersuchenden Verbindung und Bestimmen eines Toxizitätsprofils der Gallensäure, wobei das Gallensäure-Toxizitätsprofil eine toxische Wirkungsstärke umfasst; und
e) Vergleichen der toxischen Wirkungsstärke der Gallensäure zwischen den Schritten c) und d), um das Potential der Verbindung systemische und/oder hepatische Toxizität zu verursachen zu bestimmen;
wobei eine Zunahme der toxischen Wirkungsstärke in Schritt d) im Vergleich zu Schritt c) auf eine Fähigkeit eine cholestatische Hepatotoxizität hervorzurufen hinweist,
wobei eine Abnahme der toxischen Wirkungsstärke in Schritt (d) im Vergleich zu Schritt c) auf eine Fähigkeit Cholestase zu verursachen hinweist, die zu systemischer Toxizität führt, und
wobei das Bestimmen des Potentials der Verbindung systemische und/oder hepatische Toxizität zu verursachen (1) das Charakterisieren einer Verbindung als potenziell cholestatische hepatische Toxizität verursachend umfasst, wenn die Verbindung einen Anstieg der toxischen Wirkungsstärke der Gallensäure verursacht; und (2) das Bestimmen, ob die Verbindung ein Gallensäure-Efflux-Inhibitor, ein Farnesoid-X-Rezeptor (FXR) Antagonist oder eine Kombination davon ist, umfasst.

2. Verfahren nach Anspruch 1, wobei das Bestimmen des Potentials der Verbindung systemische und/oder hepatische Toxizität zu verursachen ferner umfasst, keine Änderung der toxischen Wirkungsstärke der Gallensäure bei Anwesenheit der Verbindung im Vergleich zur Abwesenheit der Verbindung zu identifizieren, einen Anstieg der toxischen Wirkungsstärke der Gallensäure bei Anwesenheit der Verbindung im Vergleich zur Abwesenheit der Verbindung zu identifizieren, oder eine Abnahme der toxischen Wirkungsstärke der Gallensäure bei Anwesenheit der Verbindung im Vergleich zur Abwesenheit der Verbindung zu identifizieren.

3. Verfahren nach Anspruch 2, wobei eine Verbindung welche keine Änderung der toxischen Wirkungsstärke der Gallensäure hervorruft als system ische oder hepatische Toxizität nicht verursachend charakterisiert wird.

4. Verfahren nach Anspruch 1, wobei die cholestatische hepatische Toxizität durch eine Hemmung des Gallensäure-Effluxes und/oder des Gallensalz-Exportproteins (BSEP) verursacht wird, wobei die Verbindung als ein Gallensäure-Efflux-Inhibitor charakterisiert wird, und/oder die cholestatische hepatische Toxizität durch den Antagonismus des Farnesoid-X-Rezeptors (FXR) verursacht wird, wobei die Verbindung als ein FXR-Antagonist charakterisiert wird, und/oder eine Kombination aus beiden.

5. Verfahren nach Anspruch 2, wobei eine eine Abnahme der toxischen Wirkungsstärke der Gallensäure verursachende Verbindung als eine Fähigkeit eine zu systemischer Toxizität führende Cholestase hervorzurufen aufweisend charakterisiert wird, wobei die zu systemischer Toxizität führende Cholestase durch Hemmung der Aufnahme von Gallensäure hervorgerufen wird.

6. Verfahren nach einem der Ansprüche 1 bis 5, wobei die Bestimmung der Neigung der Verbindung, system ische oder hepatische Toxizität hervorzurufen, die Charakterisierung umfasst, ob die Verbindung ein Inhibitor von Gallensäureaufnahme ist.

7. Verfahren nach einem der Ansprüche 1 bis 6, wobei die Verbindung ein Arzneimittelkandidat ist, wobei der Arzneimittelkandidat dadurch charakterisiert ist, dass er mit geringer Wahrscheinlichkeit systemische und/oder hepatische Toxizität verursacht, oder mit hoher Wahrscheinlichkeit systemische und/oder hepatische Toxizität verursacht.

8. Verfahre nach einem der Ansprüche 1 bis 7, wobei Gallensäuretransport Gallensäureaufnahme, basolaterale Ausscheidung und/oder kanalikuläre Ausscheidung umfasst.

9. Verfahren nach einem der Ansprüche 1 bis 8, wobei die Reihe der Konzentrationen der Gallensäure intrazelluläre Konzentrationen aufweist, die das intrazelluläre Fasten in vivo nachahmen und/oder postprandiale Konzentrationen, und/oder wobei die Reihe der Konzentrationen der Gallensäure eine ausreichende intrazelluläre Gallensäurekonzentration aufweist, um einen FXR-Rückkopplungsmechanismus zu aktivieren.

10. Verfahren nach einem der Ansprüche 1 bis 9, wobei das Bestimmen eines Toxizitätsprofils der Gallensäure das Messen einer hepatotoxischen Reaktion mittels eines Zytotoxizitätstests umfasst, wobei der Zytotoxizitätstest aus der Gruppe bestehend aus einem Enzym-Leckage-Test, ATP, APOTOX Glo und einer Kombination daraus ausgewählt wird, wobei der Enzym-Leckage-Test aus der Gruppe bestehend aus ALT, AST und LDG ausgewählt wird.

11. Verfahren nach einem der Ansprüche 1 bis 10, ferner umfassend Aussetzen des HCS einer Vielzahl an Gallensäuren, um Toxizitätsprofile für die Vielzahl der Gallensäuren zu bestimmen, wobei die Gallensäure-Toxizitätsprofile eine Vielzahl an toxischen Wirkungsstärken umfassen.

12. Verfahren nach Anspruch 11, wobei die Vielzahl an toxischen Wirkungsstärken im HCS unter Abwesenheit und Anwesenheit der Verbindung gemessen werden, um ein hepatotoxisches Potential der Verbindung vorherzusagen.

13. Verfahren nach einem der Ansprüche 1 bis 12, wobei das HCS eine zweidimensionale Kultur oder eine dreidimensionale Kultur umfasst, unter Verwendung primärer Hepatozyten oder anderer Leberzellsysteme, wobei die zweidimensionale Kultur eine Sandwich-Kultur von Hepatozyten (SHC) umfasst, wobei die SHC menschliche Leberzellen umfasst, wobei die dreidimensionale Kultur eine 3D-Kultur auf Gerüstbasis oder eine sphäroidische Kultur umfasst.

14. Verfahren nach einem der Ansprüche 1 bis 13, wobei die Verbindung dem HCS über einen Bereich von Konzentrationen ausgesetzt wird.

15. Verfahren nach einem der Ansprüche 1 bis 14, wobei die Gallensäure oder Gallensäuren GCA, GCDCA, DCA, CA, CDCA, TCA, TCDCA, LCA, GLCA, TLCA oder irgendeine Kombination daraus ist/sind, wobei eine oder mehrere freie Fettsäuren und/oder eine vorbestimmte Glukosekonzentration im HCS angewendet wird.

## Revendications

1. Une méthode pour dépister un composé susceptible de provoquer une toxicité systémique et/ou hépatique, la méthode comprenant les étapes suivantes :
(a) la fourniture d'un composé à dépister ;
(b) l'établissement d'un système cellulaire hépatique (HCS) présentant une capacité de synthèse de l'acide biliaire, de transport de l'acide biliaire et/ou de régulation de l'acide biliaire ;
(c) l'exposition du HCS à une variété de concentrations d'acide pour déterminer un profil de toxicité de l'acide biliaire, le profil de toxicité de l'acide biliaire comprenant une puissance de toxicité ; et
(d) l'exposition du HCS à une variété de concentrations de l'acide biliaire en présence du composé à dépister et la détermination d'un profil de toxicité de l'acide biliaire, le profil de toxicité de l'acide biliaire comprenant une puissance de toxicité ; et
(e) la comparaison de la puissance de toxicité de l'acide biliaire entre les étapes (c) et (d) pour déterminer le potentiel du composé de provoquer une toxicité systémique et/ou hépatique,
une augmentation de la puissance de toxicité à l'étape (d) par rapport à l'étape (c) indiquant un potentiel de provoquer une hépatotoxicité cholestatique,
une diminution de la puissance de toxicité à l'étape (d) par rapport à l'étape (c) indiquant un potentiel de provoquer une cholestase entraînant une toxicité systémique, et
la détermination du potentiel du composé de provoquer une toxicité systémique et/ou hépatique consistant à (1) caractériser un composé comme présentant un potentiel de provoquer une hépatotoxicité cholestatique si le composé entraîne une augmentation de la puissance de toxicité de l'acide biliaire ; et (2) déterminer si le composé est un inhibiteur d'écoulement d'acide biliaire, un antagoniste de récepteur farnésoïde X (FXR), ou une combinaison de ces derniers.

2. La méthode selon la revendication 1, la détermination du potentiel du composé de provoquer une toxicité systémique et/ou hépatique consistant également à identifier l'absence de changement dans la puissance de toxicité de l'acide biliaire en présence du composé par rapport à l'absence du composé, l'identification d'une augmentation de la toxicité de l'acide biliaire en présence du composé par rapport à l'absence du composé, ou l'identification d'une diminution de la toxicité de l'acide biliaire en présence du composé par rapport à l'absence du composé.

3. La méthode selon la revendication 2, un composé ne provoquant aucun changement dans la puissance de toxicité de l'acide biliaire étant caractérisé comme ne provoquant pas de toxicité systémique ou hépatique.

4. La méthode selon la revendication 1, la toxicité hépatique cholestatique étant provoquée par l'inhibition de l'écoulement d'acide biliaire et/ou une protéine d'export de sel biliaire (BSEP), le composé étant caractérisé comme un inhibiteur d'écoulement d'acide biliaire, et/ou la toxicité hépatique cholestatique étant provoquée par l'antagonisme d'un récepteur farnésoïde X (FXR), le composé étant caractérisé comme un antagoniste de FXR, et/ou une combinaison des deux.

5. La méthode selon la revendication 2, un composé provoquant une diminution de la puissance de toxicité de l'acide biliaire étant caractérisé comme présentant le potentiel de provoquer une cholestase entraînant une toxicité systémique, la cholestase entraînant une toxicité systémique étant provoquée par l'inhibition de l'absorption d'acide biliaire.

6. La méthode selon l'une quelconque des revendications 1-5, la détermination de la susceptibilité du composé de provoquer une toxicité systémique ou hépatique consistant à déterminer si le composé est un inhibiteur de l'absorption d'acide biliaire.

7. La méthode selon l'une quelconque des revendications 1-6, le composé étant un médicament candidat, le médicament candidat étant caractérisé comme présentant une faible probabilité de provoquer une toxicité systémique et/ou hépatique, ou une probabilité élevée de provoquer une toxicité systémique et/ou hépatique.

8. La méthode selon l'une quelconque des revendications 1-7, le transport de l'acide biliaire comprenant l'absorption d'acide biliaire, l'excrétion basolatérale et/ou l'excrétion canaliculaire.

9. La méthode selon l'une quelconque des revendications 1-8, l'éventail des concentrations de l'acide biliaire comprenant des concentrations intracellulaires imitant des concentrations in vivo de jeûne intracellulaire et/ou postprandiales, et/ou l'éventail de concentrations de l'acide biliaire comprenant une concentration intracellulaire d'acide biliaire suffisante pour activer un mécanisme de rétroaction du FXR.

10. La méthode selon l'une quelconque des revendications 1-9, la détermination d'un profil de toxicité de l'acide biliaire consistant à mesurer une réponse hépatotoxique à l'aide d'un test de cytotoxicité, le test de cytotoxicité étant choisi dans le groupe comprenant un test de fuite d'enzymes, l'ATP ou l'APOTOX Glo et une combinaison de ces derniers, le test de fuite d'enzymes étant choisi dans le groupe comprenant l'ALT, l'AST et la LDH.

11. La méthode selon l'une quelconque des revendications 1-10, comprenant également l'exposition du HCS à une pluralité d'acides biliaires pour déterminer les profils de toxicité pour la pluralité des acides biliaires, les profils de toxicité des acides biliaires comprenant une pluralité de puissances de toxicité.

12. La méthode selon la revendication 11, la pluralité des puissances de toxicité étant mesurée en HCS en l'absence et en présence du composé pour prédire un potentiel hépatotoxique du composé.

13. La méthode selon l'une quelconque des revendications 1-12, le HCS comprenant une culture bidimensionnelle ou tridimensionnelle, utilisant des hépatocytes primaires ou d'autres systèmes cellulaires hépatiques, la culture bidimensionnelle comprenant une culture sandwich d'hépatocytes (SCH), la SCH comprenant des hépatocytes humains, la culture tridimensionnelle comprenant une culture 3D en échafaudage ou une culture sphéroïdale.

14. La méthode selon l'une quelconque des revendications 1-13, le composé étant exposé au HCS selon un éventail de concentrations.

15. La méthode selon l'une quelconque des revendications 1-14, l'acide biliaire ou les acides biliaires étant du GCA, du GCDCA, du GDCA, du DCA, du CA, du CDCA, du TCA, du TCDCA, du LCA, du GLCA, du TLCA ou une combinaison quelconque de ces derniers, un ou plusieurs acides gras libres et/ou une concentration prédéterminée de glucose étant utilisée dans le HCS.
